# EUROPEAN PATENT SPECIFICATION

(11) **EP 4 000 527 B1**
(45) Date of publication and mention of the grant of the patent: **09.04.2025**
(21) Application number: 20306364.9
(22) Date of filing: 12.11.2020
(51) Int. Cl.: A61B 6/10, A61B 6/00, A61B 6/58

(54) **METHOD FOR OPTIMIZING A TRAJECTORY OF A MOTORIZED C-ARM**
VERFAHREN ZUR OPTIMIERUNG EINER TRAJEKTORIE EINES MOTORISIERTEN C-BOGENS
PROCÉDÉ D'OPTIMISATION D'UNE TRAJECTOIRE D'UN ARCEAU MOTORISÉ

(43) Date of publication of application: 25.05.2022
(73) Proprietor: Ecential Robotics, 38610 Gieres (FR)
(72) Inventor: ARMAND, David, 38120 Saint Egreve (FR); PIERRE, Arnaud, 38700 La Tronche (FR); LAVALLEE, Stéphane, 38140 Saint Martin d'Uriage (FR)
(74) Representative: Regimbeau

(56) References cited:
- US-A1- 2001 054 695
- US-A1- 2003 099 328
- US-A1- 2008 031 413
- US-A1- 2011 085 637
- US-A1- 2018 368 791
- US-A1- 2020 305 813

## Description

### FIELD OF THE INVENTION

The present invention relates to a method for optimizing a trajectory of a motorized C-arm.

### BACKGROUND OF THE INVENTION

X-Ray imaging systems are frequently used during medical surgical procedures to provide physicians with image-based information about a patient's anatomical situation and/or the position and orientation of surgical instruments.

Such X-ray imaging systems typically provide two-dimensional (2D) projection images with different anatomical structures superimposed along the path of the X-rays.

A typical example of such a system for use in an intra-operative setting is the so-called C-arm which comprises a base frame on which a C-shaped arm is attached with several intermediate joints allowing moving the C-shaped arm in space along several degrees of freedom. One end of the C-shaped arm carries an X-ray source and the other end carries an image detector.

Due to the limited information provided by these 2D images, three-dimensional (3D) imaging techniques have become necessary over the past decades.

While computer tomography is a well-established class of stationary X-ray imaging systems used for 3D reconstruction in a radiology department, these systems are in general not usable inside an operating room.

Recent years have seen an increasing interest in tomographic reconstruction techniques, also known as cone-beam reconstruction techniques (CBCT), using two-dimensional detectors.

Special efforts have been made to enable the aforementioned C-arms to provide three-dimensional information by automatically acquiring a set of 2D images and subsequently reconstructing a 3D image reconstruction based on said cone-beam reconstruction techniques.

An inherent problem with this imaging modality is the limitation in size of the reconstructed 3D image. The factors limiting this size of reconstruction are notably:
- the surface of the detection panel on the C-arm;
- the characteristic dimensions of the C-arm, which include in particular the distance between the X-ray source and the detection panel;
- the range of mobility of the C-arm along its degrees of freedom;
- the low number of degrees of freedom in the mobility of the C-arm and/or its components (detection panel notably), restraining the complexity of the trajectory of the C-arm for 2D images acquisitions.

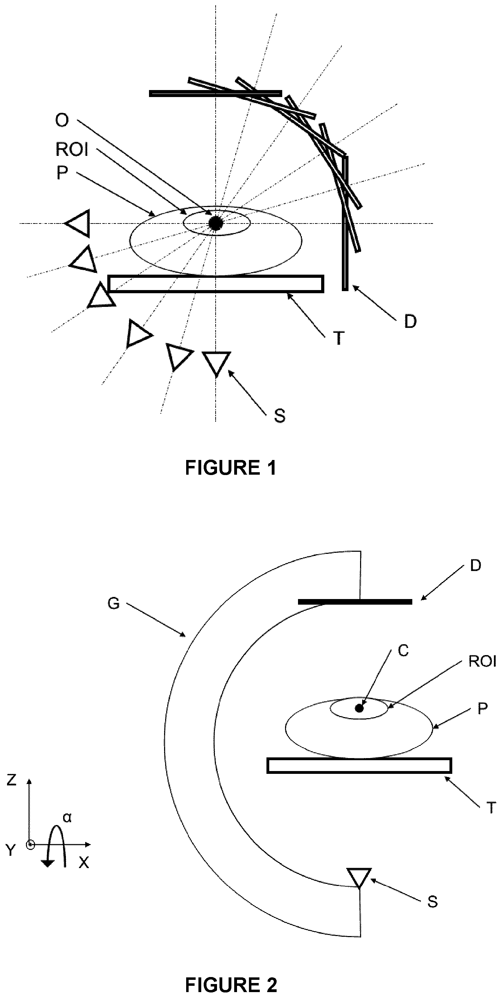

FIG. 1 is a schematic representation of an orbital trajectory of a C-arm as commonly practiced in the prior art. Several positions of the X-ray source S and the detector D along half the orbital trajectory (i.e. along a 90° angular sector) are represented, the dotted lines linking one position of the X-ray source S to the corresponding position of the detector D representing the axis of revolution of the X-ray emission cone of the X-ray source during the acquisition of a 2D X-ray image. The patient P is lying on a table T. The trajectory of the C-arm is orbital, meaning that the C-arm rotates about an axis which is perpendicular to the plane of the C-arm (which corresponds to the plane of the sheet on which FIG. 1 is drawn), over a 180° angular sector. In said plane, the center O of rotation of the C-arm is the isocenter of the C-arm, which is the center of the segment which links the X-ray source S to the detector panel D. To reconstruct a 3D image of a region of interest ROI of the patient, a plurality of 2D X-ray images of the region of interest are acquired during the orbital trajectory of the C-arm. As shown in FIG. 1, the center of rotation O of the C-arm coincides at every time with the center of the region of interest.

However, it is often desirable, during minimally invasive surgery, to have a reconstructed 3D image as large as possible, not only for the surgeon's comfort but also for better outputs in certain surgeries: for example for interventions on several bones (several vertebrae for example), or for surgery on obese patients.

US2003099328A1 discloses a C-arm system wherein, after a patient image is obtained, the C-arm is moved to the next angular scan position and an image receptor is moved radially toward or away from the patient.

### SUMMARY OF THE INVENTION

It is thus desirable to optimize the trajectory of a C-arm imaging system during the acquisition of a set of 2D images in view of generating a reconstructed 3D image of a region of interest (ROI) of a patient as large as possible within safety parameters.

The invention thus provides a method for optimizing a trajectory of a motorized C-arm for an acquisition of a 3D image of a region of interest of a body lying on an operating table, said C-arm comprising an X-ray source and an X-ray image detector, said trajectory comprising at least two different angular positions of acquisition around a rotation axis of the C-arm, said method comprising the following steps:
- determining a center of the region of interest, and
- for each angular position of the C-arm of said trajectory, computing a translation of the C-arm along a central axis extending between the X-ray source and a center of the X-ray image detector and passing by said center of the region of interest to reduce a distance between the X-ray image detector and the center of the region of interest whilst avoiding collisions between the X-ray source and detector and the operating table and/or the body.

Each 2D image corresponds to a conical projection of the region of interest onto the the X-ray detector. The size of the reconstructed 3D volume is related to the size of the intersection of every cone of projection of a 3D acquisition. Thus, as the C-arm trajectory is such that each cone of projection meets the region of interest at its largest, i.e. if the detector is as close as possible of said region of interest, the result will be an optimal reconstructed volume in terms of size.

In addition, since the region of interest is farther from the X-ray source, the X-ray dose received by the patient is reduced.

In some embodiments, a maximum displacement of the C-arm along said translation axis is reached along at least one degree of freedom of said C-arm.

In some embodiments, the trajectory is an orbital rotation, and a center of rotation of said orbital rotation follows a trajectory made of three consecutive linear segments:
- a downward vertical translation,
- a horizontal translation,
- an upward vertical translation.

In some embodiments, the trajectory comprises an orbital rotation and a rotation relative to a vertical plane transversal to the operating table.

In some embodiments, the operating table presents at least one motorized degree of freedom according to a vertical translation and the method further comprises computing a vertical translation of the operating table to reduce the distance between the X-ray image detector and the center of the region of interest.

Another object of the invention is a method for acquiring a 3D image of a region of interest of a body lying on an operating table with a motorized C-arm, comprising:
- determining an optimized trajectory of the C-arm as described above,
- controlling the C-arm to execute said optimized trajectory and acquire a set of 2D X-ray image for each respective angular position of the C-arm along said trajectory, and
- reconstructing the 3D image of the region of interest based on said set of 2D X-ray images.

In some embodiments, the C-arm comprises an anti-collision system, the method further comprising, before acquiring 2D X-ray images, activating the anti-collision system and moving the C-arm according to the optimized trajectory to detect a risk of collision with the operating table, the patient or another obstacle along said trajectory.

In some embodiments, the operating table presents at least one motorized degree of freedom according to a vertical translation, the method further comprising controlling a motorized vertical translation of the operating table during execution of the C-arm trajectory to translate the center of the region of interest toward the image detector.

Another object of the invention is an X-ray imaging system for a 3D acquisition of a region of interest of a body, comprising:
- a motorized C-arm comprising an X-ray source and an X-ray detector,
- a control unit configured to control the motorized C-arm to execute a trajectory for acquisition of a set of 2D X-ray images,
wherein the control unit is configured to compute an optimal trajectory of the motorized C-arm comprising at least an orbital rotation of the C-arm and a translation of the C-arm along a central axis extending between the X-ray source and a center of the X-ray image detector and passing by a center of the region of interest to reduce a distance between the X-ray image detector and the center of the region of interest.

In some embodiments, the X-ray imaging system further comprises an operating table presenting at least one motorized degree of freedom according to a vertical translation.

In some embodiments, the control unit is configured to synchronously control the motorized C-arm and the motorized operating table.

### BRIEF DESCRIPTION OF THE FIGURES

Further features and advantages of the invention will be described in the following description, based on the appended drawings wherein:
- FIG. 1 is a schematic representation of an orbital trajectory of a C-arm as commonly practiced in the prior art;
- FIG. 2 is a schematic representation of an X-ray imaging system that may be used in the present invention;
- FIG. 3 is a schematic view of a translation of the C-arm to reduce the distance between the region of interest and the detector;
- FIG. 4 is a schematic view of a translation of the C-arm to avoid an obstacle;
- FIG. 5 is a schematic representation of the operation of an X-ray imaging system according to an embodiment comprising a servo-controlled operating table;
- FIG. 6 is a schematic representation of the trajectory of the center of rotation of orbital motion of the C-arm gantry according to a first embodiment of the invention;
- FIG. 7 is a schematic representation of the trajectory of the center of rotation of orbital motion of the C-arm gantry according to a second embodiment of the invention;
- FIG. 8A is a schematic representation of the trajectory of the center of rotation of orbital motion of the C-arm gantry according to a third embodiment of the invention;
- FIG. 8B is a schematic representation of the trajectory of the center of rotation of orbital motion of the C-arm gantry according to a fourth embodiment of the invention;
- FIG. 9 is a schematic representation of the intersection of the cones of projection during a standard orbital trajectory as commonly practiced in the prior art;
- FIG. 10A is a schematic representation of the intersection of the cones of projection during an optimized trajectory according to an embodiment of the invention;
- FIG. 10B is a schematic representation of the intersection of the cones of projection during an optimized trajectory according to an alternative embodiment of the invention.

### DETAILED DESCRIPTION OF EMBODIMENTS

The invention may be implemented for acquiring 2D or 3D X-ray images in the context of a surgical intervention carried out onto a patient's bone, including but not limited to: implantation of orthopedic implants such as pedicular screws in the spine, implantation of various orthopedic implants in bones, reduction and fixation of fractures during traumatological procedures, positioning guides or canulae at a desired position with respect to a predefined target, or insertion of catheters or stents during cardio-vascular or urology procedures.

In this regard, the X-ray imaging system may be coupled to other surgical systems, such as a localization system and/or a surgical robotic system.

### X-ray imaging system

As depicted in FIG. 2, the X-ray imaging system comprises at least one X-ray source S and at least one X-ray image detector D. The X-ray image detector may comprise a flat detector panel.

The X-ray source and X-ray image detector are carried by a C-shaped gantry G, the X-ray source and X-ray image detector being arranged on opposite ends of the gantry. Due to the shape of the gantry, such an imaging system is usually called a C-arm. As mentioned above, the center of the segment connecting the center of the X-ray source and the center of the detector is called the isocenter of the C-arm.

In the present invention, as in various conventional C-arms, the center of orbital motion of the C-arm is the isocenter. As a result, in the present text, the center of orbital motion and the isocenter designate the same point referred to as O.

In a manner known *per se,* the X-ray imaging system is configured to produce at least one 2D X-ray image that is the result of a conical projection of a patient anatomy, wherein the tip of the cone is approximately the central point of the X-ray source and the basis of the cone is approximately the portion of the X-ray image detector that is reached by X-ray beams that have been collimated in a given shape and orientation. For example, the X-ray imaging system may be a conventional C-arm, or any Cone-Beam Computed Tomography (CBCT) imaging system such as the SURGIVISIO device (SURGIVISIO, Gières, France), or VISION FD VARIO 3D (ZIEHM), CIOS SPIN MOBILE 3D (SIEMENS), AIRO (STRYKER), LOOP-X (BRAINLAB), O-ARM (MEDTRONIC).

A conventional C-arm is designed to allow the gantry to move relative to a base so as to move the X-ray source and detector about a patient while obtaining projection images of the patient placed between the X-ray source and the X-ray detector of the gantry.

In preferred embodiments, the X-ray imaging system may be motorized. In particular, the C-shaped gantry may comprise motors allowing movement horizontally (X and Y directions), vertically (Z direction) and around the X direction (defined by an angle α), so that 2D X-ray images of the patient may be produced from almost any angle. As shown on FIG. 2, the X axis is transversal to the operating table T on which the patient lies and the Y axis is parallel to the longitudinal axis of said operating table.

Each motor is associated to an encoder that provides at any time the relative position of the X-ray imaging system with respect to a reference position. When a 2D X-ray image is acquired, the corresponding position of the imaging system is recorded. Thus, each 2D image is recorded in the referential of the imaging system.

The trajectory of the motorized C-arm is determined by each 2D X-ray image position of acquisition of said C-arm while performing a 3D image acquisition.

In some embodiments, the C-arm may comprise a mobile base (not shown) allowing displacing the C-arm in the operating room. The C-shaped gantry may thus be slidably and/or pivotably mounted on said mobile base. The motors of the C-arm may be arranged in the mobile base and/or in the gantry.

### Control unit

The X-ray imaging system is controlled by a control unit which typically comprises a processor, a data storage device and a communication device.

The control unit may advantageously be embedded in the mobile base of the X-ray imaging system. Said base may also comprise switches, such as a power switch, an emergency button and the like.

Alternatively, said control unit may be embedded in a separate cart with at least one interface with the C-arm, or may be remote, for example in a separate control room of the hospital or in a data center.

In preferred embodiments, the control unit may be able to control other surgical systems in the operating room.

### Operating table

During a surgical intervention, a patient P lies on an operating table T.

For example, in case of spine surgery, the patient may lie face down on the operating table, such that the region of interest ROI, which may comprise one or several vertebrae, is accessible to the surgeon.

In some embodiments, the operating table may be fixed relative to the ground of the operating room.

In other embodiments, the operating table may have at least one degree of freedom relative to the ground of the operating room. Preferably, said at least one degree of freedom is a translation. For example, as explained in more detail below, the operating table may be able to translate upwards or downwards along the vertical axis Z. To that end, the operating table may comprise at least one motor arranged in a foot of the table. Each motor is associated to an encoder that provides at any time the relative position of the table with respect to a reference position.

In preferred embodiments, translations of the operating table may be controlled by the control unit of the C-arm. Thus, when a 2D X-ray image is acquired, not only the corresponding position of the C-arm is recorded, but also the corresponding position of the table.

### Determination of the center of the region of interest

The determination of the optimal trajectory may comprise a first step of determining the center C of the region of interest ROI of the body to be imaged.

In some embodiments, a tool such as a set of radiopaque fiducials in a known spatial configuration may be used to this effect. It may be disposed within the volume to be imaged and in such a way that the spatial relationship between said radiopaque fiducials (or any other fixed reference) and ROI center is known and fixed.

Alternatively, the ROI center may be defined by an input of the surgeon of an operator on preoperative images or on intraoperative images acquired prior to the acquisition path for the 3D image. For example, two 2D X-ray images may be acquired with the X-ray imaging system, allowing for a 3D positioning of the ROI center. For example, said two images may be a frontal and a lateral view of the region of interest to be imaged.

In other embodiments, the center of the region of interest may be determined automatically by image analysis.

In any case, the position of the center of the region of interest may be determined in the referential of the X-ray imaging system. In such a way, the control unit is able to move the C-arm with respect to the center of the region of interest.

### Determination of the trajectory of the C-arm

In conventional image acquisitions, the C-arm operates an orbital rotation, the center of which is the isocenter of the C-arm.

In the present invention, the C-arm implements a complex trajectory, which combines a rotation about the isocenter and a translation along a central axis extending between the X-ray source and the center of the X-ray image detector and passing by the center of the region of interest to reduce or even minimize the distance between the detector and the center of the region of interest. This trajectory also takes into account the position of the operating table and the patient to avoid any collision.

In some embodiments, the complex trajectory may also include a rotation about the X-axis (α angle). Such a rotation may allow reducing the impact of metallic artefacts in the X-ray images. For example, the angle α with respect to the vertical XZ plane may vary between -5° and +5°, either continuously or discontinuously.

Thanks to this optimized trajectory, the distance between the patient's body and the X-ray image detector may be reduced as compared to a simple orbital trajectory. This provides at least two advantages:
(1) increasing the size of the 3D medical image; and
(2) decreasing the X-ray dose received by the patient.

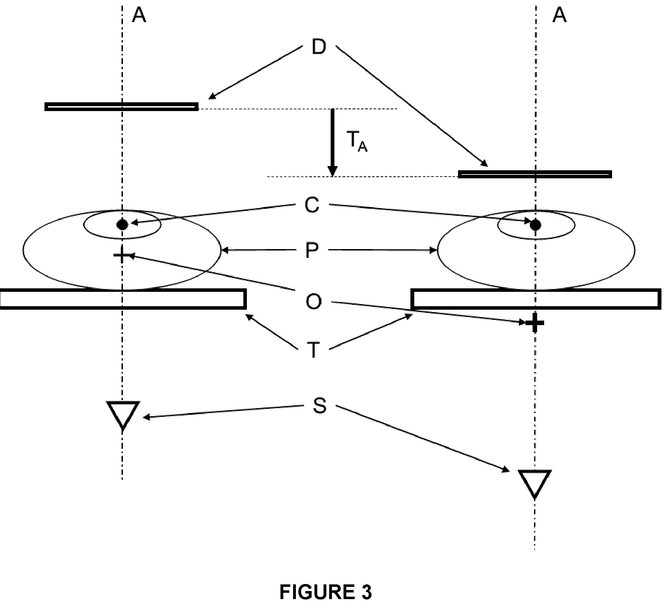

This will be best understood based on FIG. 3, which illustrates one angular position of the C-arm for an acquisition of a 2D X-ray image.

In a first step which is represented on the left side, the operating table T, patient P, X-ray source S and X-ray image detector D during an orbital movement of the C-arm are represented. The orbital center of rotation of the C-arm (which corresponds to the isocenter of the C-arm) is designated by reference O and the center of the region of interest is designated by reference C. In the illustrated situation the orbital center of rotation O is located slightly below the center C of the region of interest. The axis A represents the central axis extending between the X-ray source and the center of the X-ray image detector and passing through the center C of the region of interest.

In a second step which is represented on the right side of FIG. 3, a translation T_{A} of the C-arm along the axis A is carried out. This translation T_{A} has the effect of bringing the detector D closer to the patient. As a result, the 2D X-ray image acquired in this position of the C-arm is larger than in the situation illustrated on the left side of FIG. 3. In addition, since the X-ray source is farther from the patient, the X-ray dose received by the patient during this acquisition is lower than in the situation illustrated on the left side of FIG. 3.

The translation T_{A} is computed by the control unit to minimize the distance between the X-ray detector and the center C of the region of interest.

It should be noted that the first and second steps may be performed successively or simultaneously.

Although not shown, the C-arm may also be inclined by an angle α relative to the vertical XZ plane. This additional movement may be implemented simultaneously with the first and second steps, or as a third step following the first and second steps.

The optimized trajectory is composed of a plurality of positions of the C-arm relative to the patient, each position being determined as explained above.

Said trajectory may first be computed for the whole set of positions allowing acquiring a set of 2D X-ray images, and then be implemented so as to acquire each 2D X-ray image.

After the acquisition of a 2D X-ray image, the C-arm may not be moved directly to the next computed position, but it may be translated back to a reference position in order to avoid any collision with the patient or operating table when moving to the next acquisition position.

Of course, the control unit may also take into account the position of the patient and of the operating table (and of any other surgical system located in the vicinity of the patient) relative to the C-arm to avoid any collision.

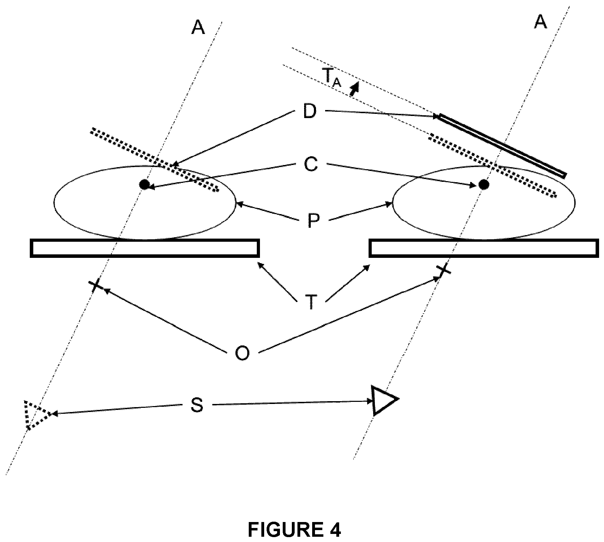

For example, FIG. 4 illustrates a situation where a collision is likely to occur between the detector D and the patient (left side of the figure). In such case, the control unit computes a translation T_{A} of the C-arm sufficient to avoid the collision while still minimizing the distance between the patient and the detector (right side of the figure).

To that end, an anti-collision system may be integrated to the X-ray imaging system. Such an anti-collision system may comprise sensors, such as proximity sensors, telemeters, tactile sensors, etc., configured to detect the presence of an object at a determined distance from at least one moving part of the C-arm. The control unit may be configured to stop the movement of the C-arm in case a sensor has detected an object at a short distance from a moving part of the C-arm.

For example, a collision avoidance trajectory may be done before the actual acquisition trajectory, with the anti-collision system enabled to detect any risk of collision with an external item along each translation computed for the optimization of the acquisition. Thus, it may be ensured that the calculated trajectory does not imply any risk of collision, while being optimal regarding the translation (i.e. the maximum translation considering the risks of collision has been performed). If this collision avoidance trajectory allows confirming that there is no risk of collision, the acquisition trajectory may be implemented accordingly.

Of course, the anti-collision system may remain activated during the acquisition trajectory, in order to avoid any collision in the case unexpected obstacles are present along the trajectory.

In some situations, the translation of the C-arm may be limited by the maximum range of motion of the motors. For example, the trajectory of the C-arm may not be able to fully minimize the distance between the region of interest and the detector due to the fact that the motors have reached their maximum positions and cannot further translate the C-arm.

To avoid this situation, the user of the C-arm may be advised to begin the trajectory with all motors of the C-arm positioned in the middle of their range of motion.

However, this precaution may not be sufficient and it may happen than the C-arm cannot be translated sufficiently to minimize the distance between the detector and the center of the region of interest.

In such case, an operating table having at least one degree of freedom may allow compensating the limits of the motors of the C-arm and contributing to the minimization of the distance between the detector and the center of the region of interest. According to a preferred embodiment, said degree of freedom is a translation along a vertical direction.

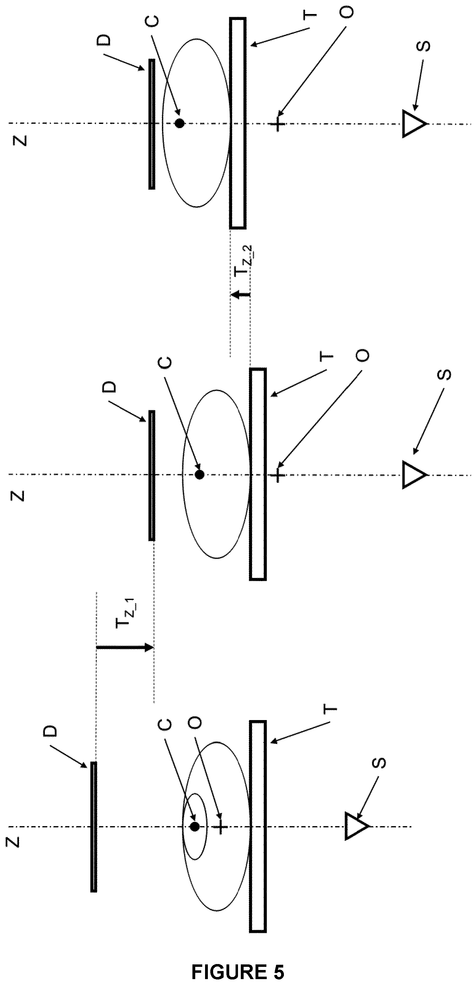

Such an embodiment is illustrated in FIG. 5.

On the left side of the figure, the C-arm is positioned along a vertical direction Z.

In the middle of the figure, the C-arm may be moved downwards in the vertical direction by a translation T_{Z_1}, with the effect of bringing the detector as close as possible to the center C of the region of interest. However, due to a limitation in the range of motion of the corresponding motors of the C-arm, said distance is not minimal.

In order to minimize said distance, the operating table may be moved upwards in the vertical direction by a translation T_{Z_2}. As shown in the right side of the figure, the distance between the detector and the region of interest may thus be minimized.

Once the acquisition of the 2D X-ray image has been performed, the operating table may return to a lower reference position to avoid any collision with the C-arm.

Preferably, the translation of the C-arm and of the operating table are both controlled by the control unit, which allows a synchronous operation of both devices.

When considering the trajectory of the center of rotation of the orbital motion of the C-arm as a whole, one may consider that it has a substantially U shape. By U shape is meant a planar shape made of three consecutive segments oriented substantially at 90° relative to each other. The segments may be substantially linear, but may alternatively have a certain radius of curvature. In addition, the connection between the segments may be a right angle or may present a small radius of curvature. According to a preferred embodiment, first and third segments extend in a vertical direction and the second segment which connects the first and third segments extends in a horizontal direction. The second segment is advantageously connected to the bottom end of the first and third segments.

This kind of trajectory has proven to be an optimal trade-off between maximization of the volume and avoidance of any risk of collision.

The U shape may be rectangular or square, which may be obtained by not using more than one degree of freedom of the C-arm gantry (apart from the orbital motion) for the translation along the central X-ray axis for a given acquisition position.

Alternatively, the U shape may be round for an optimal but more complex trajectory involving at least two degrees of freedom of the C-arm gantry (apart from the orbital motion) for both reconstructed volume size and dose received by the patient.

The one of more degrees of freedom of the C-arm gantry may be selected taking into account the acquisition time and the mechanical capabilities of the C-arm.

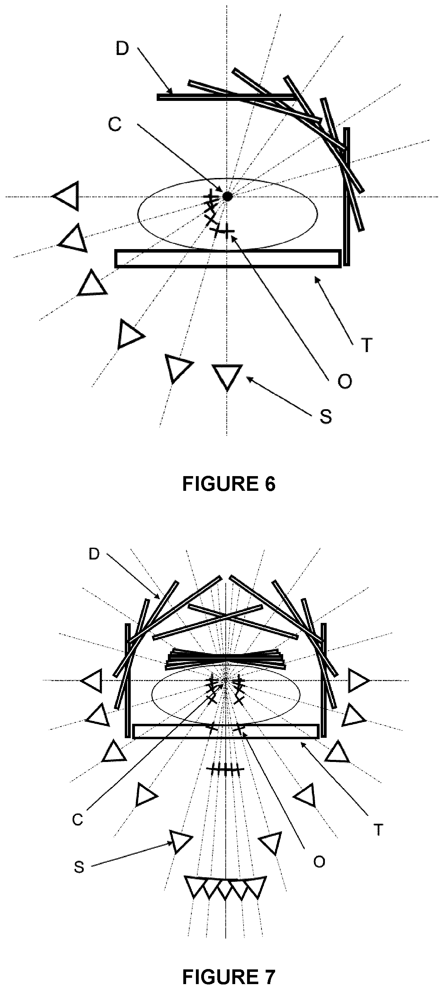

In FIG. 6, several positions of the X-ray source and X-ray detector during half a trajectory (90° angular rotation) of the C-arm are represented, with the dotted lines corresponding to the central axis extending between the X-ray source and the center of the X-ray image detector and passing by the center of the region of interest for each angular position of the C-arm. The plurality of positions of the center O of the orbital motion of the C-arm are arranged around the center C of the region of interest according to half a square U shape.

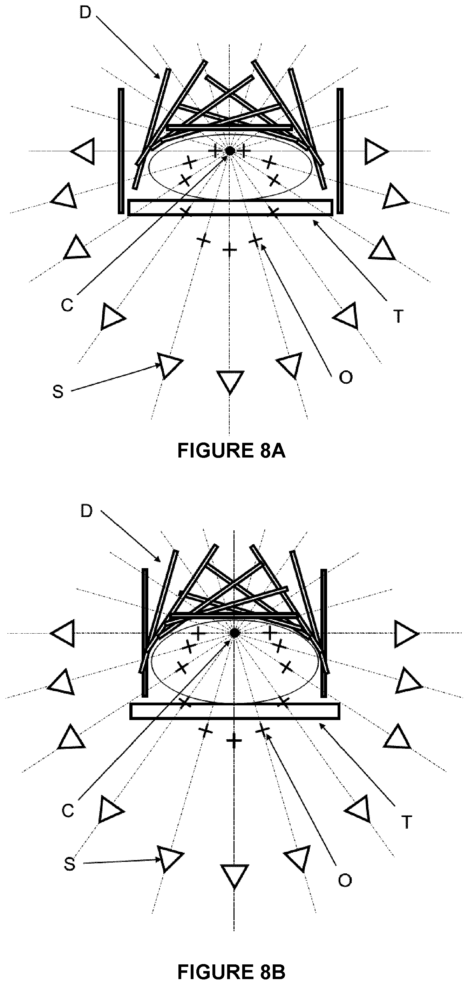

FIGS. 7 and 8 illustrate alternative U-shaped trajectories of the C-arm.

In the embodiment of FIG. 7, the translation of the C-arm is computed so as to minimize the distance between the X-ray detector and the center of the region of interest. The trajectory of the orbital center of the C-arm has substantially a rectangular U shape. In this case, the vertical translation of the C-arm is computed to move the detector as close as possible to the patient's body, but the detector remains farther from the patient's body when inclined relative to the vertical direction. In this way, the X-ray dose received by the patient is minimized. This embodiment requires a large range of motion of the motors controlling the vertical translation of the C-arm. As mentioned above, this embodiment may also be achieved using an operating table movable along the vertical direction.

In the embodiment of FIG. 8A, the trajectory of the center O of rotation of the orbital motion of the C-arm has a round U shape. In each acquisition position of the C-arm, the distance between the detector and the center of the region of interest is minimized.

The embodiment of FIG. 8B differs from the one of FIG. 8A in that the patient is more corpulent, which results in the fact that the center C of the region of interest is located farther from the operating table T. As a result, according to the optimized trajectory, the translation of the detector D toward the center C is not hindered by the operating table, which allows placing the detector D closer to the patient along the whole trajectory, especially laterally.

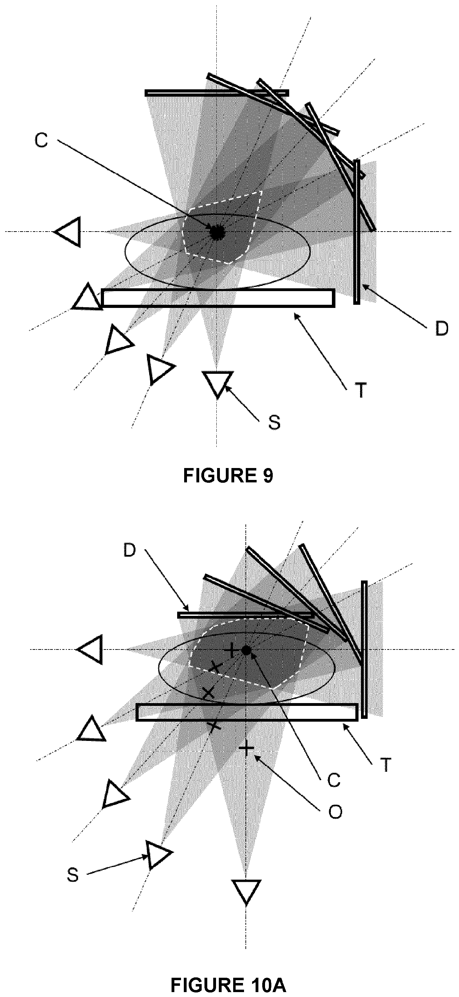

FIGS. 9 and 10A-10B illustrate how the size of the 3D image may be increased thanks to the trajectory according to the invention.

FIG. 9 is a schematic representation of the intersection of the cones of projection during a standard orbital trajectory as commonly practiced in the prior art. Each cone of projection extending from the source to the detector is represented by a light grey triangle; the image, which corresponds to the intersection of all the cones of projection, is represented by the darkest color. Its limits are indicated by the white dotted line.

FIG. 10A is a schematic representation of the intersection of the cones of projection during an optimized trajectory according to the invention. As in FIG. 9, each cone of projection is represented by a light grey triangle; the image, which corresponds to the intersection of all the cones of projection, is represented by the darkest color. Its limits are indicated by the white dotted line. The size of the image in FIG. 10A is clearly greater than the size of the image in FIG. 9.

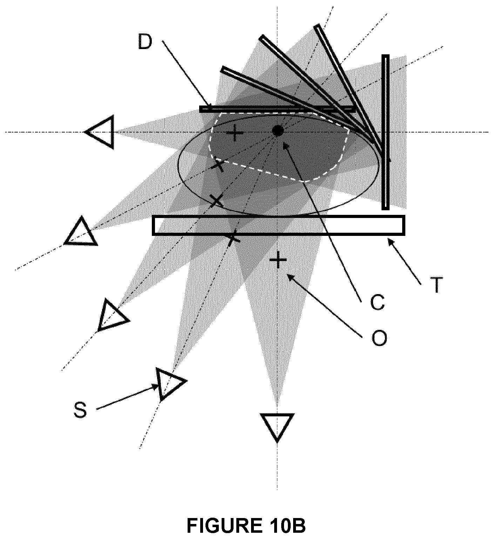

The embodiment of FIG. 10B differs from the one of FIG. 10A in that the patient is more corpulent, which results in the fact that the center C of the region of interest is located farther from the operating table T. As a result, according to the optimized trajectory, the translation of the detector D toward the center C is not hindered by the operating table, which allows placing the detector D closer to the patient along the whole trajectory, especially laterally.

Of course, the shape of the trajectory represented in the drawings is only illustrative and not limitative.

## Claims

1. Method for optimizing a trajectory of a motorized C-arm for an acquisition of a 3D image of a region of interest (ROI) of a body (P) lying on an operating table (T), said C-arm comprising an X-ray source (S) and an X-ray image detector (D), said trajectory comprising at least two different angular positions of acquisition around a rotation axis of the C-arm, said method being **characterized in that** it comprises:
- determining a center (C) of the region of interest (ROI) in a referential of the C-arm;
- for each angular position of the C-arm of said trajectory, computing a translation (T_{A}, T_{Z_1}) of the C-arm along a central axis extending between the X-ray source (S) and a center of the X-ray image detector (D) and passing by said center (C) of the region of interest to reduce a distance between the X-ray image detector (D) and the center (C) of the region of interest whilst avoiding collisions between the X-ray source and detector and the operating table (T) and/or the body (P).

2. Method according to claim 1, wherein a maximum displacement of the C-arm along said translation axis is reached along at least one degree of freedom of said C-arm.

3. Method according to any one of claims 1 or 2, wherein the trajectory is an orbital rotation about an axis (Y) perpendicular to a plane of the C-arm, and a center (O) of rotation of said orbital rotation follows a trajectory made of three consecutive linear segments:
- a downward vertical translation along an axis (Z) perpendicular to the operating table,
- a horizontal translation,
- an upward vertical translation.

4. Method according to any one of claims 1 or 2, wherein the trajectory comprises an orbital rotation about an axis (Y) perpendicular to a plane of the C-arm and a rotation (α) relative to a vertical (XZ) plane transversal to the operating table (T), said vertical plane (XZ) being perpendicular to the operating table.

5. Method according to any one of claims 1 to 4, wherein the operating table (T) presents at least one motorized degree of freedom according to a vertical translation along an axis (Z) perpendicular to the operating table and the method further comprises computing a vertical translation (T_{Z_2}) of the operating table to reduce the distance between the X-ray image detector (D) and the center (C) of the region of interest.

6. Method for acquiring a 3D image of a region of interest (ROI) of a body (P) lying on an operating table (T) with a motorized C-arm, comprising:
- determining an optimized trajectory of the C-arm according to any one of claims 1 to 5, and
- controlling the C-arm to execute said optimized trajectory and acquire a set of 2D X-ray images for each respective angular position of the C-arm along said optimized trajectory, and
- reconstructing a 3D image of the region of interest based on said set of 2D X-ray images.

7. Method according to claim 6, wherein the C-arm comprises an anti-collision system, the method further comprising, before acquiring 2D X-ray images, activating the anti-collision system and moving the C-arm according to the optimized trajectory to detect a risk of collision with the operating table, the patient or another obstacle along said trajectory.

8. Method according to any one of claims 6 to 7, wherein the operating table presents at least one motorized degree of freedom according to a vertical translation, the method further comprising controlling a motorized vertical translation (T_{Z_2}) of the operating table during execution of the C-arm trajectory to translate the center (C) of the region of interest toward the image detector (D).

9. X-ray imaging system for a 3D acquisition of a region of interest of a body, comprising:
- a motorized C-arm comprising an X-ray source (S) and an X-ray detector (D),
- a control unit configured to control the motorized C-arm to execute a trajectory for acquisition of a set of 2D X-ray images,
wherein the control unit is configured to compute an optimal trajectory of the motorized C-arm comprising at least an orbital rotation of the C-arm about an axis (Y) perpendicular to a plane of the C-arm and a translation of the C-arm along a central axis extending between the X-ray source (S) and a center of the X-ray image detector (D) and passing by a center (C) of the region of interest to reduce a distance between the X-ray image detector and the center of the region of interest.

10. X-ray imaging system according to claim 9, further comprising an operating table presenting at least one motorized degree of freedom according to a vertical translation along an axis (Z) perpendicular to the operating table.

11. X-ray imaging system according to claim 10, wherein the control unit is configured to synchronously control the motorized C-arm and the motorized operating table.

## Patentansprüche

1. Verfahren zum Optimieren einer Trajektorie eines motorisierten C-Bogens für eine Erfassung eines 3D-Bildes eines interessierenden Bereichs (ROI) eines Körpers (P), der auf einem Operationstisch (T) liegt, wobei der C-Bogen eine Röntgenquelle (S) und einen Röntgenbilddetektor (D) umfasst, wobei die Trajektorie mindestens zwei unterschiedliche Winkelpositionen zur Erfassung um eine Drehachse des C-Bogens herum umfasst, wobei das Verfahren **dadurch gekennzeichnet ist, dass** es umfasst:
- Bestimmen eines Mittelpunkts (C) des interessierenden Bereichs (ROI) in einem Bezugssystem des C-Bogens;
- Berechnen einer Verschiebung (T_{A}, T_{Z_1}) des C-Bogens entlang einer Mittelachse, die sich zwischen der Röntgenquelle (S) und einem Mittelpunkt des Röntgenbilddetektors (D) erstreckt und durch den Mittelpunkt (C) des interessierenden Bereichs verläuft, für jede Winkelposition der Trajektorie des C-Bogens, um einen Abstand zwischen dem Röntgenbilddetektor (D) und dem Mittelpunkt (C) des interessierenden Bereichs zu verringern und zugleich Kollisionen zwischen der Röntgenquelle und dem Detektor und dem Operationstisch (T) und/oder dem Körper (P) zu vermeiden.

2. Verfahren nach Anspruch 1, wobei eine maximale Verlagerung des C-Bogens entlang der Verschiebungsachse entlang mindestens eines Freiheitsgrades des C-Bogens erreicht wird.

3. Verfahren nach einem der Ansprüche 1 oder 2, wobei die Trajektorie eine Bahndrehung um eine Achse (Y) senkrecht zu einer Ebene des C-Bogens ist, und ein Drehmittelpunkt (O) der Bahndrehung einer Trajektorie folgt, die aus drei aufeinanderfolgenden linearen Segmenten besteht:
- einer vertikalen Verschiebung nach unten entlang einer Achse (Z) senkrecht zum Operationstisch,
- einer horizontalen Verschiebung,
- einer vertikalen Verschiebung nach oben.

4. Verfahren nach einem der Ansprüche 1 oder 2, wobei die Trajektorie eine Bahndrehung um eine Achse (Y) senkrecht zu einer Ebene des C-Bogens und eine Drehung (α) relativ zu einer vertikalen (XZ) Ebene quer zum Operationstisch (T) umfasst, wobei die vertikale Ebene (XZ) zum Operationstisch senkrecht ist.

5. Verfahren nach einem der Ansprüche 1 bis 4, wobei der Operationstisch (T) mindestens einen motorisierten Freiheitsgrad gemäß einer vertikalen Verschiebung entlang einer Achse (Z) senkrecht zum Operationstisch aufweist und das Verfahren weiter das Berechnen einer vertikalen Verschiebung (T_{Z_2}) des Operationstisches umfasst, um den Abstand zwischen dem Röntgenbilddetektor (D) und dem Mittelpunkt (C) des interessierenden Bereichs zu verringern.

6. Verfahren zum Erfassen eines 3D-Bildes eines interessierenden Bereichs (ROI) eines Körpers (P), der auf einem Operationstisch (T) liegt, mit einem motorisierten C-Bogen, umfassend:
- Bestimmen einer optimierten Trajektorie des C-Bogens nach einem der Ansprüche 1 bis 5, und
- Steuern des C-Bogens, um die optimierte Trajektorie auszuführen und einen Satz 2D-Röntgenbilder für jede jeweilige Winkelposition des C-Bogens entlang der optimierten Trajektorie zu erfassen, und
- Rekonstruieren eines 3D-Bildes des interessierenden Bereichs auf Basis des Satzes 2D-Röntgenbilder.

7. Verfahren nach Anspruch 6, wobei der C-Bogen ein Kollisionsschutzsystem umfasst, wobei das Verfahren vor dem Erfassen von 2D-Röntgenbildern weiter das Aktivieren des Kollisionsschutzsystems und das Bewegen des C-Bogens gemäß der optimierten Trajektorie umfasst, um eine Kollisionsgefahr mit dem Operationstisch, dem Patienten oder einem anderen Hindernis entlang der Trajektorie zu erkennen.

8. Verfahren nach einem der Ansprüche 6 bis 7, wobei der Operationstisch mindestens einen motorisierten Freiheitsgrad gemäß einer vertikalen Verschiebung aufweist, wobei das Verfahren weiter das Steuern einer motorisierten vertikalen Verschiebung (T_{Z_2}) des Operationstisches während der Ausführung der C-Bogen-Trajektorie umfasst, um den Mittelpunkt (C) des interessierenden Bereichs zum Bilddetektor (D) hin zu verschieben.

9. Röntgenbildgebungssystem für eine 3D-Erfassung eines interessierenden Bereichs eines Körpers, umfassend:
- einen motorisierten C-Bogen, der eine Röntgenquelle (S) und einen Röntgendetektor (D) umfasst,
- eine Steuereinheit, die dazu konfiguriert ist, den motorisierten C-Bogen so zu steuern, dass er eine Trajektorie zur Erfassung eines Satzes 2D-Röntgenbilder ausführt,
wobei die Steuereinheit dazu konfiguriert ist, eine optimale Trajektorie des motorisierten C-Bogens zu berechnen, die mindestens eine Bahndrehung des C-Bogens um eine Achse (Y) senkrecht zu einer Ebene des C-Bogens und eine Verschiebung des C-Bogens entlang einer Mittelachse, die sich zwischen der Röntgenquelle (S) und einem Mittelpunkt des Röntgenbilddetektors (D) erstreckt und durch einen Mittelpunkt (C) des interessierenden Bereichs verläuft, umfasst, um einen Abstand zwischen dem Röntgenbilddetektor und dem Mittelpunkt des interessierenden Bereichs zu verringern.

10. Röntgenbildgebungssystem nach Anspruch 9, das weiter einen Operationstisch umfasst, der mindestens einen motorisierten Freiheitsgrad gemäß einer vertikalen Verschiebung entlang einer Achse (Z) senkrecht zum Operationstisch aufweist.

11. Röntgenbildgebungssystem nach Anspruch 10, wobei die Steuereinheit dazu konfiguriert ist, den motorisierten C-Bogen und den motorisierten Operationstisch synchron zu steuern.

## Revendications

1. Méthode d'optimisation d'une trajectoire d'un arceau motorisé pour une acquisition d'une image 3D d'une région d'intérêt (ROI) d'un corps (P) allongé sur une table d'opération (T), ledit arceau comprenant une source de rayons X (S) et un détecteur d'images par rayons X (D), ladite trajectoire comprenant au moins deux positions angulaires d'acquisition différentes autour d'un axe de rotation de l'arceau, ladite méthode étant **caractérisée en ce qu'**elle comprend :
- déterminer un centre (C) de la région d'intérêt (ROI) dans un référentiel de l'arceau ;
- pour chaque position angulaire de l'arceau de ladite trajectoire, calculer une translation (T_{A}, T_{Z_1}) de l'arceau le long d'un axe central s'étendant entre la source de rayons X (S) et un centre du détecteur d'images par rayons X (D) et passant par ledit centre (C) de la région d'intérêt afin de réduire une distance entre le détecteur d'images par rayons X (D) et le centre (C) de la région d'intérêt tout en évitant les collisions entre la source de rayons X et le détecteur et la table d'opération (T) et/ou le corps (P).

2. Méthode selon la revendication 1, dans laquelle un déplacement maximal de l'arceau le long de l'axe de translation est atteint le long d'au moins un degré de liberté dudit arceau.

3. Méthode selon l'une quelconque des revendications 1 ou 2, dans laquelle la trajectoire est une rotation orbitale autour d'un axe (Y) perpendiculaire à un plan de l'arceau, et un centre (O) de rotation de ladite rotation orbitale suit une trajectoire constituée de trois segments linéaires consécutifs :
- une translation verticale vers le bas le long d'un axe (Z) perpendiculaire à la table d'opération,
- une translation horizontale,
- une translation verticale vers le haut.

4. Méthode selon l'une quelconque des revendications 1 ou 2, dans laquelle la trajectoire comprend une rotation orbitale autour d'un axe (Y) perpendiculaire à un plan de l'arceau et une rotation (α) par rapport à un plan vertical (XZ) transversal à la table d'opération (T), ledit plan vertical (XZ) étant perpendiculaire à la table d'opération.

5. Méthode selon l'une des revendications 1 à 4, dans laquelle la table d'opération (T) présente au moins un degré de liberté motorisé selon une translation verticale le long d'un axe (Z) perpendiculaire à la table d'opération et ma méthode comprend en outre le calcul d'une translation verticale (T_{(Z_2})) de la table d'opération pour réduire la distance entre le détecteur d'images par rayons X (D) et le centre (C) de la région d'intérêt.

6. Méthode d'acquisition d'une image 3D d'une région d'intérêt (ROI) d'un corps (P) allongé sur une table d'opération (T) à l'aide d'un arceau motorisé, comprenant :
- déterminer une trajectoire optimisée de l'arceau selon l'une des revendications 1 à 5, et
- commander l'arceau pour exécuter ladite trajectoire optimisée et acquérir un ensemble d'images 2D par rayons X pour chaque position angulaire respective de l'arceau le long de ladite trajectoire optimisée, et
- reconstruire une image 3D de la région d'intérêt sur la base de cet ensemble d'images 2D par rayons X.

7. Méthode selon la revendication 6, dans laquelle l'arceau comprend un système anti-collision, la méthode comprenant en outre, avant l'acquisition d'images par rayons X 2D, l'activation du système anti-collision et le déplacement de l'arceau selon la trajectoire optimisée pour détecter un risque de collision avec la table d'opération, le patient ou un autre obstacle le long de ladite trajectoire.

8. Méthode selon l'une des revendications 6 à 7, dans laquelle la table d'opération présente au moins un degré de liberté motorisé selon une translation verticale, la méthode comprenant en outre la commande d'une translation verticale motorisée (T_{Z_2}) de la table d'opération pendant l'exécution de la trajectoire de l'arceau pour déplacer le centre (C) de la région d'intérêt vers le détecteur d'images (D).

9. Système d'imagerie à rayons X pour l'acquisition en 3D d'une région d'intérêt d'un corps, comprenant :
- un arceau motorisé comprenant une source de rayons X (S) et un détecteur de rayons X (D),
- une unité de commande configurée pour contrôler l'arceau motorisé afin d'exécuter une trajectoire pour l'acquisition d'un ensemble d'images 2D par rayons X,
dans lequel l'unité de commande est configurée pour calculer une trajectoire optimale de l'arceau motorisé comprenant au moins une rotation orbitale de l'arceau autour d'un axe (Y) perpendiculaire à un plan de l'arceau et une translation de l'arceau le long d'un axe central s'étendant entre la source de rayons X (S) et un centre du détecteur d'images par rayons X (D) et passant par un centre (C) de la région d'intérêt afin de réduire la distance entre le détecteur d'images par rayons X et le centre de la région d'intérêt.

10. Système d'imagerie à rayons X selon la revendication 9, comprenant en outre une table d'opération présentant au moins un degré de liberté motorisé selon une translation verticale le long d'un axe (Z) perpendiculaire à la table d'opération.

11. Système d'imagerie à rayons X selon la revendication 10, dans lequel l'unité de commande est configurée pour commander de manière synchrone l'arceau motorisé et la table d'opération motorisée.
